## EUROPEAN PATENT SPECIFICATION

(45) Date of publication and mention
of the grant of the patent:
**02.11.2022 Bulletin 2022/44**

(21) Application number: **13819389.1**

(22) Date of filing: **18.07.2013**

(51) International Patent Classification (IPC):
**A61Q 17/00** (2006.01)    **A61Q 19/00** (2006.01)
**A61K 8/9789** (2017.01)    **A23L 3/3472** (2006.01)
**A61K 36/87** (2006.01)    **A23L 33/105** (2016.01)

(52) Cooperative Patent Classification (CPC):
**A61K 36/87; A23L 3/3472; A23L 33/105;
A61K 8/9789; A61Q 17/005; A61Q 19/00;**
A61K 2236/00; A61K 2800/524

(86) International application number:
**PCT/ES2013/070526**

(87) International publication number:
**WO 2014/013122 (23.01.2014 Gazette 2014/04)**

(54) **POLYPHENOL EXTRACT FROM WHITE-GRAPE RESIDUE**

POLYPHENOLEXTRAKT AUS WEISSER TRAUBENMAISCHE

EXTRAIT POLYPHÉNOLIQUE OBTENU À PARTIR DE RÉSIDUS DE RAISIN BLANC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.07.2012 ES 201231152**

(43) Date of publication of application:
**27.05.2015 Bulletin 2015/22**

(73) Proprietor: **Universidade De Santiago De
Compostela
15782 Santiago de Compostela - A Coruña (ES)**

(72) Inventors:
- **LORES AGUIN, Marta**
 **E-15782 Santiago de Compostela La Coruña (ES)**
- **GARCIA JARES, Carmen**
 **E-15782 Santiago de Compostela La Coruña (ES)**
- **ALVAREZ CASAS, Marta**
 **E-15782 Santiago de Compostela La Coruña (ES)**
- **LLOMPART, Maria**
 **E-15782 Santiago de Compostela La Coruña (ES)**

(74) Representative: **Teixeira de Carvalho, Anabela
Patentree
Edificio Net
Rua de Salazares 842
4149-002 Porto (PT)**

(56) References cited:
**ES-A1- 2 217 966    US-B1- 6 238 673**

- **MAKRIS ET AL: "Polyphenolic content and in
vitro antioxidant characteristics of wine industry
and other agri-food solid waste extracts",
JOURNAL OF FOOD COMPOSITION AND
ANALYSIS, ACADEMIC PRESS, LONDON, GB,
vol. 20, no. 2, 24 November 2006 (2006-11-24),
pages 125-132, XP005745299, ISSN: 0889-1575,
DOI: 10.1016/J.JFCA.2006.04.010**
- **MAKRIS ET AL: "Recovery of antioxidant
phenolics from white vinification solid
by-products employing water/ethanol mixtures",
BIORESOURCE TECHNOLOGY, ELSEVIER BV,
GB, vol. 98, no. 15, 1 May 2007 (2007-05-01), pages
2963-2967, XP022036064, ISSN: 0960-8524, DOI:
10.1016/J.BIORTECH.2006.10.003**
- **MARIVEL S PRG A!NCHEZ ET AL: "Antioxidant
power, bacteriostatic activity, and
characterization of white grape pomace extracts
by HPLC-ESI-MS", EUROPEAN FOOD
RESEARCH AND TECHNOLOGY ; ZEITSCHRIFT
FÜR LEBENSMITTELUNTERSUCHUNG UND
-FORSCHUNG A, SPRINGER, BERLIN, DE, vol.
230, no. 2, 24 October 2009 (2009-10-24), pages
291-301, XP019756784, ISSN: 1438-2385, DOI:
10.1007/S00217-009-1177-Y**

- PEINADO J ET AL: "Antioxidant activity of different phenolics fractions isolated in must from Pedro Ximenez grapes at different stages of the off-vine drying process", FOOD CHEMISTRY, ELSEVIER LTD, NL, vol. 114, no. 3, 1 June 2009 (2009-06-01), pages 1050-1055, XP025938119, ISSN: 0308-8146, DOI: 10.1016/J.FOODCHEM.2008.10.068 [retrieved on 2008-11-06]
- JONGBERG S. ET AL.: 'Effect of white grape extract and modified atmosphere packaging on lipid andprotein oxidation in chill stored beef patties.' FOOD CHEMISTRY vol. 128, 2011, pages 276 - 283, XP028198933

**Description**

**Field of the invention**

The present invention relates to polyphenolic extracts, their procedures for obtaining them and their uses.

**Background of the invention**

Barker *et al.* developed a new method for disruption and extraction of solid samples: the matrix solid-phase dispersion (MSPD) (US 08/026,275). This procedure combines aspects of several techniques for the disruption of the sample and, simultaneously, generates a material having a unique character to extract the compounds of interest from a given sample. The first applications of MSPD were targeted to the isolation of active components or metabolites in biological matrices, but subsequently this technique has also been applied to the extraction of natural compounds (vitamins, flavonoids), pesticides and other pollutants from animal tissues, fruits, vegetables and other food, biological and environmental matrices.

In the original conception, MSPD involved the mixing of a viscous, solid or semisolid sample with a solid silica support previously derivatized to produce a linked organic phase such as octadecylsilyl ($C_{18}$) on its surface. Some applications of MSPD have involved mixing the samples with underivatized silicates (silica gel, sand, etc.) or other organic solids (graphitized fibers) or inorganic (Florisil, alumina, etc.) (Barker, S.A. Matrix solid-phase dispersion (MSPD). J. Biochem. Biophys. Methods, 2007, 70, 151-162).

The first applications of the MSPD to the extraction of polyphenols were in the field of medicinal plants, specifically phenolic acids (Ziakova A, Brandsteterová E, Blahová E (2003) Matrix solid-phase dispersion for the liquid chromatographic determination of phenolic acids in Melissa officinalis, Journal of Chromatography A 983, 271-275) and isoflavonoids (Xiao HB, Krucker M, Albert K, Liang XM (2004) Determination and identification of isoflavonoids in Radix astragali by matrix solid-phase dispersion extraction and high-performance liquid chromatography with photodiode array and mass spectrometric detection, Journal of Chromatography A 1032, 117-124). Later, Manhita *et al* applied it to the extraction of anthocyanins from vegetable samples *(*Journal of Chromatography A, 2006, 1129, 14-20), in what appears to be the first application of this technique in *Vitis vinifera* (red grapes).

Occasionally, available amount of samples for testing are limited, particularly in the case of biological samples, so that errors in the sample preparation stage become unacceptable, and protocols involving the use of small amounts of sample become very valuable. Thus, the MSPD technique has been optimized for extractions with very small amounts of samples, less than 50 milligrams and using an amount of solvent in the order of microliters, what makes this technique in a miniaturized process that allows the chromatographic analysis of samples (Kristenson, E.M.; Haverkate, E.G.J.; Slooten, C.J.; Ramos, L.; Vreuls, R.J.J.; Brinkman, U.A.Th. Miniaturized automated matrix solid-phase dispersion extraction of pesticides in fruit followed by gas chromatographic-mass spectrometric analysis. J. Chromatogr. A 2001, 917, 277-286; Kristenson, E.M.; Shahmiri, S.; Slooten, C.J.; Vreuls, R.J.J.; Brinkman, U.A.Th. Matrix solid-phase dispersion micro-extraction of pesticides from single insects with subsequent GC-MS analysis. Chromatographia 2004, 59, 315-320).

Document XPOO5745299: "Polyphenolic content and in vitro antioxidant characteristics of wine industry and other agri-food solid waste extracts", discloses an extract from white grape and the extraction process of polyphenols from white grape pomace with sand and methanol, acetone and water as solvent.

Document US6238673B1, discloses a method of manufacturing a dry polyphenol composition, the method including contacting liquid grape extract, using red grapes, with a nonoxidizing chromatographic separation medium which fractionates the components of the extract; eluting material from the separation medium with an eluent; recovering that fraction in which the polyphenols are present.

Although there are various techniques for extracting polyphenols from winemaking by-products, there is still a need to provide a simple, effective and easily scalable method.

**Brief description of the invention**

The present invention provides a simple method and in few steps to obtain extracts with antioxidant and antibacterial properties from white grape by-products. This method uses non contaminant materials, it takes place in mild conditions and besides it avoids suspended solids in the obtained eluates, therefore a later filtration step is not necessary. The method of the invention allows obtaining extracts at an industrial scale.

Therefore, in one aspect the invention relates to a method, as defined in claim 1, to obtain an extract from white grape by-products comprising:

a) preparing a mixture comprising a white grape by-product and a dispersant,
b) adding a solvent,

c) eluting, and

d) collecting the eluates.

In another aspect, the invention is directed to a white grape polyphenolic extract, as defined in claim 5, characterized by the absence of anthocyans and a pH between 4 and 8 measured in aqueous solution.

In additional aspects, the invention relates to a cosmetic, pharmaceutical or nutritional composition comprising the extract previously disclosed.

Furthermore, the invention also relates to the use of the extract of the invention to prepare a food preservative or an antibacterial composition.

**Description of the figures**

Figure 1. Appearance of the extracts in solution obtained in some of the examples.

Figure 2 shows the total polyphenol index, expressed as mg gallic acid equivalents (GAE) per gram of grape marc (dry weight) relative to the mass of the starting sample (1, 10 and 20 g, respectively).

Figure 3 shows the antioxidant activity of the extracts from Example 1.1 in millimols of Trolox.

Figure 4. Test of resistance-sensitivity to the antimicrobial agent conducted with the extract of the white grape by-product obtained in the Example 1 in a plate covered by colonies of the Gram + bacteria *S. aureus.* The self-evident inhibition zone size versus control is observed.

Figure 5. Test of resistance-sensitivity to the antimicrobial agent showing the growth inhibition zones at two different dilutions of the extract (left) versus controls of the solvent used (right). a) *Bacillus spp;* b) *Saureus* ; c) *P. acnes*

Figure 6. Absence and presence of bacterial colonies in a Petri dish with the Minimum Inhibitory Concentration and the previous concentration tested of the white grape marc extract. a) *Saureus* (plate I: 1.5%; plate II: 1,25%); b) *Bacillus spp* (Plate I: 1.25 %; Plate II: 1%).

Figure 7. UV spectrum (200-400 nm) of a 1:500 dilution of the extract obtained in the example 2.

**Detailed description of the invention**

The method described above is simple, inexpensive and easily scalable. The method has been designed to extract polyphenols in such a manner that the eluates obtained have antioxidant and antibacterial properties, they do not comprise suspended solids therefore the later steps of filtration and/or ultra-filtration are not necessary. This is an additional advantage because it simplifies the method and the obtained extracts of interest.

The method of the invention allows to use any white grape marc as starting material (the distillation residue). In a particular embodiment, the white grape by-product is selected from grape marc, or exhausted grape marcs. The distribution of phenolic substances in the grapes is not homogeneous, for example the pulp contains approximately between 83 and 91%; the peels or skins between 7 and 12% and the seeds can arise 6%. In a particular embodiment, the white grape by-product is grape marc. In a particular embodiment, the white grape by-product is stored previously between -40°C and 20°C, preferably between -30°C and 10°C.

The grape is the edible fruit of the vine, small, round or oval shape with a very juicy flesh and a thin skin; it is used to make wine. Most cultivated grape comes from the species *Vitis vinifera,* natural Mediterranean Europe and Central Asia. The grapes are mainly classified into two types: white and black grapes or red.

Each type of grape has a composition in different polyphenols, particularly as it relates to white and red grapes, which will be reflected in the wines that are produced from them. In general, there are more amount of polyphenols in red wines, due to the different way in which it is fermented. While white wine is made exclusively by fermenting the juice squeezed from the grapes, red wine is made by steeping for a while the wort with the skins and pips. This makes the waste generated in the white winemaking process (white grape marc) is very rich in polyphenols, to the extent that they have passed to the wine in smaller amounts.

Therefore, white grape by-products are of interest for the present invention. The white grape by-products may come from any white grape variety. In a particular embodiment, the white grape by-products are selected from the Airen, Alarije, Albalonga, Albañá, Albariño, Cainho Branco, Aligoté, Altesse, Arinto, Arneis, Arvine, Assyrtiko, Auxerrois Blanc, Barcelos, Bogdanusa, Bacchus, Bical, Bouvier, Bual, Catarratto, Chardonnay, Chasselas, Chenin Blanc, Clairette, Cortese, Courbu, Crouchen, Cserszegi Fúszeres, Doradillo, Erbaluce, Ehrenfelser, Ezerjó, Faber, Falanghina, Fetească Alba, Fetească Regală, Fiano, Flora, Freisamer, Fromenteau, Garganega Bianca, Gewürztraminer, Gloria, Godello, Goldriesling, Greco, Grenache lanc, Grenache Gris, Grüner Veltliner, Hárslevelu, Hondarribi Zuri, Huxelrebe, Irsai Oliver, Izsáki Sarfehér, Jacquère, Juhfark, Kerner, Krstac, Len de l'El, Macabeo, Madeleine Angevine, Malvar, Malvasia, Petit Manseng, Gros Manseng, Maria Gomes, Marsanne, Mauzac, Merseguera, Moschofilero, Mtsvane, Rivaner, Muscadet de Bourgogne, Muscat, Moscatel de Málaga, de Setúbal, Moscatel Branco, Muscadelle, Muscat Ottonel, Neuberger, Ondenc, Ortega, Parc, Palomino Fino, Parellada, Pecorino, Pedro Ximénez, Pinot Blanc, Pinot Gris, Prosecco, Rabigato, Reichensteiner, Rhoditis, Riesling, Rieslaner, Rkatsiteli, Rotgipfler, Roussanne, Sauvignon Blanc, Savagnin Blanc, Savatiano, Scheurebe, Schönburger, Sémillon, Septiner, Sercial, Siegerrebe, Sylvaner, Smederevka, Spatrot, Sultana, Symphony, Torrontés, Trebbiano, Treixadura, Trousseau Gris, Verdejo, Verdiso, Verdicchio, Verduzzo Friulano, Verduzzo Trevigiano, Vermentino, Vernaccia, Viognier, Riesling Italico, Würzer, Xarello, Xynisteri, Zeta, and Zeusz variety.

In a more particular embodiment, the white grape by-products are selected from white grape by-products of Albariño, Treixadura, Godello, Loureira and Caíño Blanco variety.

The 95% of the total production of White grape in Galicia (Consello Regulador DO Rias Baixas) is the Albariño variety. In a particular embodiment of the invention, the white grape by-product is grape marc of the variety selected from the group consisting of Albariño, Caíño Blanco, Godello, Torrontés and Treixadura.

In the context of the present invention, "dispersant" relates to a solid material which mixed with a biological sample enables its disruption and dispersion on its surface, so that the mechanical mixture disturbs the sample architecture, breaking the material into smaller pieces and increasing the release of the chemical compounds present in the interior of the plant cells of the sample.

The dispersant is sand. In a particular embodiment, the dispersant is sand from a natural source. In a particular embodiment, the grain size of the dispersant is selected between 100 $\mu$m y 1 mm, preferably between 250 $\mu$m and 320 $\mu$m.

The preparation of the mixture comprising white grape by-product and a dispersant may be performed using a mortar (glass, porcelain, agate) or any other device that allows crushing and homogenizing the mixture mechanically. This step takes place at a temperature range between 10 ° C and 40 ° C and atmospheric pressure, reasons for which there is no risk of degradation of the compounds to be extracted. In a particular embodiment, the dispersant and the sample are mixed at a temperature of between 10 and 40 ° C and a pressure of between 0.8-1.2 atm. In a particular embodiment the weight ratio of the white grape by-product and the dispersant is between 1: 1 and 1: 3, preferably is 1: 2.

In a particular embodiment, the solvent of step b) disclosed above is in a proportion of between 0.05 and 8 volumes of solvent relative to the weight of the mixture of white grape by-product and dispersant, preferably between 0.1 and 5 volumes. Alternatively, it is possible to maintain for a certain time the suspension formed in step b) under certain conditions so that maceration of the sample occurs. Thus, in a particular embodiment the invention is directed to a method as described above wherein the suspension of step b) is kept between 1 and 36 hours at a temperature of between 10 °C and 40 °C. Preferably between 5 and 24 hours.

The authors of the invention found that the recirculation of the eluates collected in step d) allows decreasing the total volume of solvent used in the process and also allows significantly increasing the amount of total polyphenols extracted from white grape by-products. Thus, in a particular embodiment, the method of the invention further comprises eluting the mixture of step a) with the eluates collected in step d).

In a particular embodiment step d) is repeated between 1 and 7 times. In a particular embodiment, step d) is repeated 3, 4 or 5 times.

The combination of the steps of maceration and recirculation of eluates leads to a more optimized method in relation to the yield of the obtained extract and solvent consumption. Thus in a particular embodiment, the method of the invention comprises:

a') preparing a mixture comprising a white grape by-product and a dispersant,
b') adding a solvent to the mixture prepared in step a) and keeping between 1 and 36 hours at a temperature between 10°C and 40°C,
c') eluting the mixture of step a) with a solvent,
d') collecting the eluates,
e') eluting the mixture of step a) with the eluates collected in step d').

The solvent is selected independently from the group consisting of water, alkyl alcohol, glycols, or mixtures thereof.

In the present invention "alkyl alcohol" relates to a substance with a straight or branched hydrocarbon chain which contains a hydroxyl group, between 1 and 12 carbon atoms, preferably between 1 and 6 carbon atoms. Examples of alkyl alcohols are methanol, ethanol, isopropyl alcohol, tert-butyl alcohol, etc. In a particular embodiment, the alkyl alcohol is selected from methanol, ethanol, isopropyl alcohol, and mixtures thereof.

In the present invention "glycols" relates to a substances with a straight or branched hydrocarbon chain which contains two or more hydroxyl groups, between 1 and 12 carbon atoms, preferably between 1 and 8 carbon atoms, and optionally it may contain one or more ether groups. In a particular embodiment, the glycol is selected from ethane-1,2diol (ethylenglycol), butane-1,4-diol (1,4-butylenglycol), butane-1,3-diol, butane-1,2-diol, butane-2,3-diol, 1,5-pentanediol (pentylenglycol), 2-methyl-2,4-pentanediol (hexylenglycol), 2-(2-ethoxyethoxy)etanol (diethylenglycol monoethyl ether), 2,2'-dihydroxydipropyl ether (dipropylenglycol) and 1,2-octanodiol (caprilyl glycol), and mixtures thereof.

In a particular embodiment, the method of the invention comprises the following steps:

a") preparing a mixture comprising white grape marc and sand in a weight ratio of between 1:1 and 1:3,

b") adding a mixture of glycols/water in a volume of between 0.1 and 5 relative to the weight of the mixture of white grape by-product and dispersant in the step a") and keeping between 5 and 24 hours at a temperature between 10°C and 40°C,

c") eluting,

d") collecting the eluates,

e") eluting the mixture of step a) with the eluates collected in step d').

In a particular embodiment, the solvent used in the method of the invention can have a modified pH between 0.5 and 3. This pH is achieved by adding organic or inorganic acids to the solvent prior to its use, such as for example hydrochloric acid, acetic acid, phosphoric acid, etc.

In a particular embodiment, the method of the invention further comprises removing the solvent.

In another particular embodiment, the method of the invention comprises further a lyophilization step. In this step of lyophilization is possible to freeze previously the collected eluates or the eluates after removing the eluates. In a particular embodiment, the lyophilization is carried out between -50°C y -20°C, preferably between -45°C y - 35°C. In a particular embodiment the lyophilization is carried out between $1.31.10^{-6}$ atm and $6.60.10^{-6}$ atm (1-50 $\mu$m Hg), preferably between $1.31.10^{-6}$ atm and $1.31.10^{-5}$ atm (1-10 $\mu$m Hg). The extract is stable and the addition of stabilizers in the freeze drying step is not necessary. However, it is possible to add small amounts of sugars such as glucose, sucrose or trehalose in a concentration from 1% up to 5% or other molecules which act as cryoprotectant and/or lyoprotectant. The extract of the invention after the lyophilization is not altered in any way.

In other embodiment, the invention is directed to a white grape polyphenolic extract obtainable by any of the methods described above, as defined in claim 5.

The invention is directed to a white grape polyphenolic extract characterized by the absence of anthocyans and a pH between 4 and 8 measured in aqueous solution. The invention is directed to a white grape polyphenolic extract obtainable by the method defined in claim 1, characterized by the absence of anthocyans and a pH between 4 and 8 measured in aqueous solution.

The invention is directed to a white grape polyphenolic extract characterized by a) a total polyphenol index of between 8 and 50 mg of gallic acid/g dry grape marc of; b) gallic acid concentration of between 0.035 and 0.125 mg/g dry grape marc; c) catechin concentration of between 0.3 and 1.28 mg/g dry grape marc; d) epicatechin concentration of between 0.3 and 0.80 mg/g dry grape marc; e) pH between 4 and 8; and f) absence of anthocyans.

In a particular embodiment, the invention is directed to a white grape polyphenolic extract characterized by a) a total polyphenol index of between 15 and 25 mg of gallic acid/g dry grape marc; b) gallic acid concentration of between 0.064 and 0.082 mg/g dry grape marc; c) catechin concentration of between 0.4 and 1.23 mg/g dry grape marc; d) epicatechin concentration of between 0.63 and 0.68 mg/g dry grape marc; e) pH between 4 and 8 measured in aqueous solution; and f) absence of anthocyans.

In a particular embodiment, the extract previously described comprises further at least one compound selected from quercetins, epicatechin-gallate and procyanidins B1 and B2, and mixtures thereof. In a particular embodiment, the quercetins are selected from quercetin, quercetin-3-glucoside and quercetin-3-glucuronide.

The analytical methods and the apparatus used are described in the examples. Accordingly to the present invention, "anthocyans" refers to a group of flavonoids polyphenols which comprises the group constituted by anthocyanins and anthocyanidins, which also comprises the osidics combinations of anthocyanidins with a sugar. They are responsible for the bluish red color of the skin of red grapes.

In a particular embodiment, the white grape polyphenolic extract is obtainable by the method previously described.

In a particular embodiment, the extract is in solution, in suspension or lyophilized. In another particular embodiment, the extract is in a solid form, as powder or as a paste.

In another aspect, the invention is directed to a cosmetic composition comprising the extract previously described.

The cosmetic compositions according to the present invention include any liquid composition or any composition which comprises the extract of the invention and which is in a gel form, cream, ointment or balm for its topical administration.

Said cosmetic composition can be applied to different superficial parts of the human or animal body such as skin, hair system, nails, lips or mucous of human or animal body. In a particular embodiment, the cosmetic composition can also incorporate lypophilic or hydrophilic active molecules having properties as cosmetic or personal hygiene agent. Among the active molecules that may be incorporated can be cited emolients, preservatives, fragrance substances, anti-acne agents, antifungal agents, antioxidants, deodorants, antiperspirants, agents against dandruff, depigmenting agents, antiseborrheic agents, dyes, suntan lotions, UV light absorbers, enzymes, among others.

In other aspect, the invention refers to a pharmaceutical or dermatological composition comprising the extract previously described.

The pharmaceutical or dermatological compositions according to the invention, include any liquid composition or any composition in a gel form, ointment, cream or balm for its topical administration.

Because of its antibacterial properties, the extract of the invention is suitable for the preparation of an antibacterial composition. In an aspect, the invention refers to the use of the extract previously described for the preparation of an antibacterial composition. In a particular embodiment, the antibacterial composition is an antibacterial composition with activity against Gram-positive bacteria. In a particular embodiment, the invention refers to the use of the extract previously described for the preparation of an anti-acne composition. In a particular embodiment, the invention refers to the extract previously described for its use as anti-acne agent. In other aspect, the invention is directed to an anti-acne composition comprising the extract of the invention.

In other aspect the invention refers to a nutritional composition comprising the extract previously described. Said nutritional composition can be a food, a dietary supplement or a nutritional supplement. The nutritional compositions can include milk, yogurt, fruit and vegetables juices, desserts, products for children or dehydrated products. The addition of the extract to the nutritional composition is carried out by mixing and homogenizing according the technical procedure to prepare each product.

The extract of the invention is suitable for the preparation of a food preservative, due its antioxidant properties. In an aspect, the invention refers to the use of the extract previously described for the preparation of a food preservative. This food preservative may be a food additive or can form part of a package or biodegradable packaging. The food preservatives according to the invention include any liquid composition or any composition comprising the extract of the invention and which is in a solid form, gel or solution for its addition to food o to its package.

In other aspect, the invention is directed to a package or biodegradable packaging comprising the extract of the invention.

In other aspect, the invention is directed to a food preservative comprising the extract of the invention.

The following examples are illustrating the invention, which should not be interpreted as a limitation thereof.

## Methods

### Sample preparation and storage

The white grape marc samples used are from the following grape varieties: Albariño, Treixadura, Godello, Loureira and Caíño Blanco, autochthonous varieties from Galicia (NW Spain) coming from the winemaking wastes of wineries from the 5 Galician Appellations of Origin.

To calculate the moisture content of the grape marc samples, 3 g of grape marc were dried in an oven at 105 °C. The sample was weighed before and after the drying step. This operation was carried out in triplicate.

The grape marc was collected in each particular winery on the day of its production immediately after the pressing process, placed into plastic freezer bags ($20 \times 20$ cm), sealed, and stored at -20 °C.

For the homogenization of the samples containing skins, pulp and seeds, the grape marc was ground with a conventional electric coffee grinder before proceeding to extraction by the proposed method.

### Total Polyphenols Index (TP)

In the following examples the TP or total polyphenols index, expressed as mg gallic acid equivalents (GAE) per g of grape marc (dry weight), is measured. This is a classic and robust spectrophotometric index for which the method described by Singleton et al. was used (Singleton, V. L.; Rossi, J. A., Jr., Colorimetry of total phenolics with phosphomolybdic-phosphotungstic acid reagents. Am. J. Enol. Vitic. 1965, 16, (3), 144-58). The reaction mixture was prepared by mixing 5 mL of a Milli-Q water dilution of the extract, 100 $\mu$L of the Folin & Ciocalteu reagent and 1 mL of an aqueous solution of sodium carbonate (20% $Na_2CO$ Folin & Ciocalteu) previously prepared. After vortexing, the reaction mixture was kept in dark at room temperature for 30 min, time enough to reduce the Folin & Ciocalteu reagent by the polyphenolic compounds under alkaline conditions, resulting in the development of a blue color. The spectrometric measure was carried out at 760 nm. Milli-Q water was used as blank for the measure. TP contents were quantified from a calibration curve prepared with gallic acid standard solutions at concentrations ranging from 3 to 20 mg L$^{-1}$ (($y = 0.0735x-0.0044$: $R^2 = 0.9985$), and expressed as mg of gallic acid equivalents in the liquid extract (mg GAE L$^{-1}$). TP sample concentrations

in final extracts were expressed as mg gallic acid per g of dry weight of the grape marc sample (mg gallic g$^{-1}$ dw).

**Polyphenols Analysis by High Performance Liquid Chromatography (HPLC)**

The concentrated extracts obtained as described in examples 1 and 2, were filtered through a 0.22 $\mu$m PVDF filter and analysed by high performance liquid chromatography using a HPLC system equipped with a diode array detector (DAD); the chromatographic column used was a 3.9 mm × 150 mm, 4 $\mu$m, 60 Å, Waters Nova-Pak C18 column. The injection volume was 20 $\mu$L.

The two mobile phase solvents were (A) 1% formic acid/water and (B) 1% formic acid/methanol. The mobile phase gradient program started with 5% B, changed to 20% B at 20 min, and then changed to 100% B at 25 min. The entire HPLC run time was 25 min with a flow rate of 1.0 mL min$^{-1}$ and 50 °C column temperature.

The main polyphenols in the extract were detected at 280 nm and at 350 nm and identified by comparison of their retention times and UV spectra to those of pure standards in the same experimental conditions.

**Antioxidant Activity (AA)**

The antioxidant activity was determined using a modified method of Brand-Williams *et al.* (W. Brand-Williams, M.E. Cuvelier, C. Berset, LWT - Food Sci. Technol. 28 (1995) 25) with 2,2-diphenyl-1-picrylhydrazyl (DPPH) against Trolox®. Briefly, 0.1 mM DPPH was dissolved in 100% methanol. An aliquot (0.1 mL) of each grape marc extract was added to 3.9 mL of the methanolic DPPH solution. The mixture was shaken vigorously and allowed to stand at room temperature in the dark for 30 min. The decrease in absorbance of the resulting solution after 30 min was measured at 515 nm. The AA was calculated using the following equation (y = 0.5223x + 0.0276; R2 = 0.999), obtained from linear regression after plotting the absorbance at 515 nm ($A_{515}$) known solutions of Trolox against concentration (0.08-1 mM). The DPPH radical scavenging activity of the extracts was expressed as mM Trolox g$^{-1}$ of dry weight. The radical stock solution was prepared daily.

**Antiradical Activity**

By adapting the previous assay, also using the stable radical 2,2-diphenyl-1-picrylhydrazyl (DPPH), the half maximal inhibitory concentration (IC$_{50}$) was determined as a measure of the effectiveness of the extract for eliminating free radicals, 2 mL of a methanolic DPPH solution 6.10$^{-5}$ M are added to 50 $\mu$L of an antioxidant methanolic solution. The decrease in absorbance of the resulting solution after 16 min was measured at 515 nm ($A_{515}$) in a UV-Vis spectrophotometer. The inhibition percentage (IP) of the DPPH radical for each particular extract assayed is determined according with the following equation::

$$IP = [(A_{t = 0\,min} - A_{t = 16\,min})/A_{t = 0\,min}] \times 100$$

The IC$_{50}$ was calculated as the concentration of the grape marc extract that causes 50% inhibition of the DPPH radical. The synthetic antioxidants butylated hydroxyanisole (BHA) and butylated hydroxytoluene (BHT) have been used as reference compounds. **Example 1.** General process for the preparation of antioxidants and antibacterial extracts from white grape residues.

The extraction of Albariño grape marc is performed in a column with one frit at the bottom and another one at the top. The ratio dispersant:sample is 2:1 and the mixture is ground in a mortar. The mixture is filled into the column and it is slightly compacted for a greater contact with the eluent. Next, the elution solvent is passed ensuring a constant elution, and the eluate is collected.

A sample of 20 g mixed with 40 g of sand is crushed, and it is introduced into the glass column, long and narrow (1.5 cm diameter × 45 cm long) and lightly compacted. The volume of elution solvent, EtOH: H$_2$O (50:50), is 50 mL. In this particular embodiment, the extract was concentrated under vacuum to 8 times. The yield is 6.9% and the final extract has a total polyphenol content of 29%. Data obtained for this particular embodiment are listed in Table 1.

Table 1: Characteristic data of the extract obtained in Example 1.

| Total Polyphenols Index | 18.31 mg GAE/g dry weight |
|---|---|
| Gallic acid | 103 ppm (0.074 mg/g) |
| Catechuin | 1692 ppm (1.21 mg/g) |
| Epicatechuin | 920 ppm (0.66 mg/g) |

(continued)

| pH | 5.90 |
|---|---|

The extract is also characterized by the presence of caftaric acid, procyanidins (B1 and B2), epicatechin-gallate and quercetins (specifically, quercetin, quercetin-3-glucoside and quercetin 3-glucuronide).

**Example 1.1.** Study of the Solvent

The extraction was conducted as described in Example 1 using different solvents: aqueous methanol, aqueous ethanol, aqueous butylene glycol, and grape marc spirit; and the results based on the total polyphenols index, expressed as mg gallic acid equivalents per gram of dry grape marc (Table 2) were compared.

**Table 2:** TP data according to the type of solvent used.

| | TP (mg GAE/g dry weight) | |
|---|---|---|
| **Solvent** | Mean | standard deviation |
| MeOH:$H_2$O (80:20) | 16.08 | 0.92 |
| EtOH:$H_2$O (80:20) | 16.20 | 1.07 |
| EtOH:$H_2$O (50:50) | 18.31 | 0.25 |
| Grape marc spirit | 22.75 | 2.20 |
| Butylen glycol: $H_2$O (65:35) | 13.54 | 0.7 |

**Example 1.2.** Study of the combination dispersant-solvent

The obtainment of the extracts from white grape marc of the Albariño variety was performed as described in Example 1, using sand from different origins as the dispersant agent.

No significant differences ($P_{95}$ = 0.076) between the data obtained with commercial sand and natural sand were noticed (Table 3); nor with any combination with the solvents ($P_{95}$ = 0.15) (Table 3).

**Table 3:** TP data obtained according to the origin of the sand and the type of solvent used

| **Sand/Solvent** | TP (mg GAE/g dry weight) | | | |
|---|---|---|---|---|
| | Mean | Standard deviation | Lower limit | Upper limit |
| Natural/EtOH | 16.27 | 0.48 | 15.47 | 17.08 |
| Natural/MeOH | 16.84 | 0.59 | 15.85 | 17.82 |
| Commercial/EtOH | 16.09 | 0.21 | 15.77 | 16.44 |
| Commercial/MeOH | 15.33 | 0.47 | 15.01 | 15.64 |

Neither significant differences ($P_{95}$ = 0.48) were observed according to the grain size, after sieving natural sand through sieves of different mesh (250-320 $\mu$m) (Table 4).

**Table 4:** TP data obtained by the grain size of the natural sand.

| **Grain size** | TP (mg GAE/g dry weight) | | | |
|---|---|---|---|---|
| | Mean | Standard deviation | Lower limit | Upper limit |
| < 320 $\mu$m | 16.27 | 1.43 | 14.98 | 18.2 |
| 320-250 $\mu$m | 16.07 | 0.34 | 15.68 | 16.43 |
| < 250 $\mu$m | 17.06 | 1.17 | 15.93 | 18.2 |

**Example 1.3.** Study of maceration

The extraction was conducted as described in Example 1. The maceration stage is studied by maintaining the contact between the solvent and the mixture for three hours, to finally collect the solvent by gravity or using a pump, which accelerates the process of collecting the eluate. The pump used has a maximum flow rate of 7 L/min.

After analyzing the effect of soak or not to soak the mixture grape marc:dispersant with the solvent; and the use or not of a pump, it is concluded that it is better to macerate and to pump (Table 5).

**Table** 5: TP data obtained in the study of the combination of two factors: pump and maceration.

| Maceration/ Pump | TP (mg GAE/g dry weight) | | | |
|---|---|---|---|---|
| | Mean | Standard deviation | Lower limit | Upper limit |
| with/with | 26.02 | 0.33 | 25.76 | 26.30 |
| with/without | 27.03 | 0.19 | 26.76 | 27.30 |
| without/with | 24.03 | 0.22 | 23.77 | 24.30 |
| without/without | 17.66 | 0.21 | 17.40 | 17.93 |

**Example 1.4.** Scaled

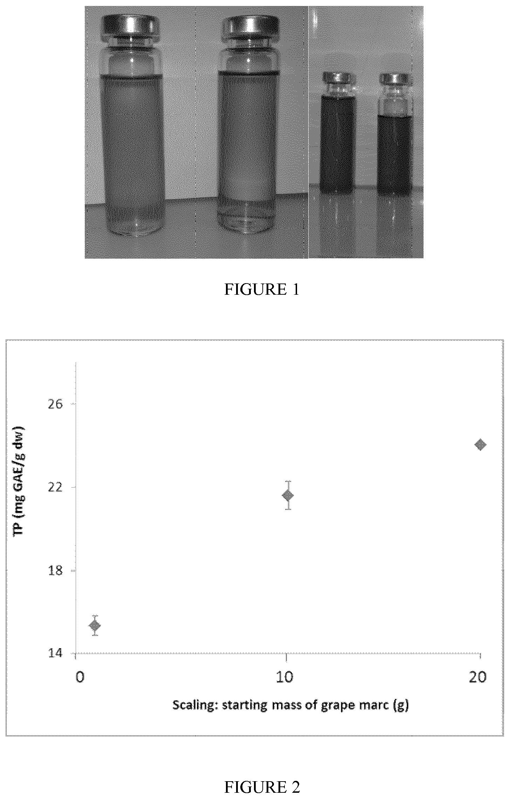

The extraction was carried out as described in Example 1, but using different initial quantities of grape marc: 1 g, 10 g and 20 g. The results demonstrate that as it increases the mass of grape marc, keeping constant the ratio sample:dispersant, the polyphenols extraction increases proportionally (Figure 2, Table 6).

**Table 6:** TP data obtained with different starting sample mass.

| Initial weight of white grape residue (g) | TP (mg GAE/g dry weight) | | | |
|---|---|---|---|---|
| | Mean | Standard deviation | Lower limit | Upper limit |
| 1 | 15.33 | 0.47 | 14.79 | 15.72 |
| 10 | 21.61 | 0.68 | 20.78 | 22.20 |
| 20 | 24.03 | 0.19 | 23.84 | 24.26 |

**Example 1.5.** Recirculation

The extraction was carried out according to Example 1, but in this case the amount of initial sample is 100 g mixed with 200 g of dispersant. For these experiments a column of 5 cm diameter × 37 cm long was used. A volume of 500 mL of a mixture EtOH:H$_2$O 50:50 was used and it was recirculated three times. Data are shown in the following table (Table 7).

**Table 7:** TP data obtained in the scaling ×100 with recirculation.

| MSPD×100 recirculation | TP (mg GAE/g dry weight) |
|---|---|
| 1st fraction (500 ml) | 16.17 |
| 2nd fraction (490 ml) | 17.06 |
| 3rd fraction (480 ml) | 21.82 |

**Example 1.6.** White grape varieties

The extraction was conducted as described in Example 1 using white grape varieties different from Albariño. Specifically the following varieties underwent the process: Caíño Blanco, Godello, Loureira and Treixadura; all varieties commonly grown for white wine production. Data are shown in the following table (Table 8).

**Table 8:** TP data obtained with the different white varieties tested.

| | TP (mg GAE/g dry weight) | |
|---|---|---|
| | Mean | Standard deviation |
| Caíño Blanco | 10.2 | 0.75 |
| Godello | 11.79 | 0.98 |
| Loureiro | 11.54 | 1.18 |
| Torrontés | 14. 57 | 0.13 |
| Treixadura | 14.31 | 1.63 |

**Example 1.7.** Seeds

The extraction was conducted as described in Example 1 using as starting raw material only the seeds isolated from white grape marc. TP of the extract obtained from the seeds is 40.21 ± 3.78 (mg GAE / g dry weight).

**Example 2. Scaling the overall process for obtaining antibacterial and antioxidant extracts from white grape residues.**

The extraction from white grape residues of Albariño variety is performed in a column with dimensions larger than those used in Example 1. In this particular embodiment, between 1 and 10 kg of grape marc are mixed with between 1 and 30 kg of dispersant. Once crushed, the mixture is filled into the column and then the eluting solvent is introduced. The mixture is macerated between 6 and 36 hours and then ensuring a constant elution, the eluate is collected. The volume of elution solvent, a mixture of 1,3-butanediol and water is between 1 and 5 L. Data obtained for this particular embodiment are listed in Table 9.

Table 9: Characteristic data of the extract obtained in Example 2.

| | mg /L | mg /g dry weight |
|---|---|---|
| **Total Polyphenols Index** | 17766[1] ± 135 | 15.63[1] ± 0.12 |
| **Gallic acid** | 99 ± 0.02 | 0.09 ± 0.00 |
| **Catechuin** | 787 ± 3 | 0.69 ± 0.00 |
| **Epicatechuin** | 809 ± 35 | 0.71 ± 0.03 |
| **Quercetins** | 45 ± 0.3 | 0.04 ± 0.00 |
| [1]miligrams GAE/g dw | | |

To assess the robustness of the procedure described in Example 2, in terms of variability, estimated as the coefficient of variation of the response variables measured, the process was performed in quintuplicate held constant the experimental conditions. The data obtained are summarized in Table 10:

Table 10: Mean values of the extracts obtained as described in Example 2 (n = 5).

| | 1 | 2 | 3 | 4 | 5 | mean | CV (%) |
|---|---|---|---|---|---|---|---|
| | mg / g dry weight | | | | | mean | CV (%) |
| IPT | 15.39[1] | 13.90[1] | 14.83[1] | 14.08[1] | 15.28[1] | 14.70 | 4.63 |
| Gálico | 0.087 | 0.087 | 0.084 | 0.074 | 0.092 | 0.085 | 7.61 |
| Catequina | 0.69 | 0.60 | 0.61 | 0.53 | 0.65 | 0.61 | 10.02 |
| Epicatequina | 0.71 | 0.60 | 0.55 | 0.51 | 0.66 | 0.61 | 13.19 |
| Quercetinas | 0.039 | 0.038 | 0.033 | 0.039 | 0.037 | 0.037 | 6.71 |
| [1]miligrams GAE | | | | | | | |

**Example 3.** Evaluation of the activities of the obtained extracts

Example 3.1. Antioxidant and anti-scavenging activity

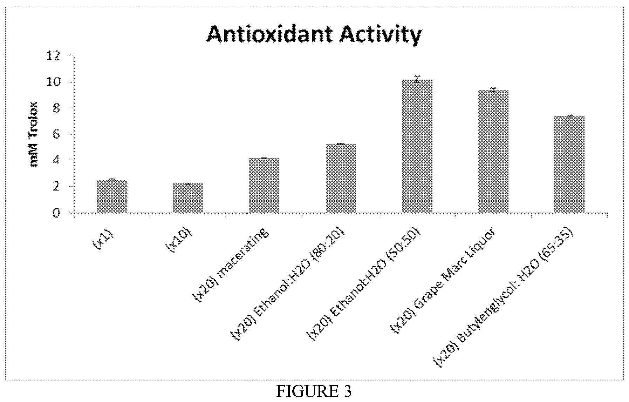

The antioxidant activity (AA) of the extracts achieved at different scales, using different combinations of solvents or using different types of initial raw material was determined, as shown in Tables 11 and 12 (and Figure 3).

In the case of changes in the pH of the elution solvent, this is set with concentrated HCl monitoring it with a pHmeter. The best results were obtained at 20 g-scale using EtOH: $H_2O$ (50:50) or grape marc spirit as solvents, and the differences observed between both results were not significant from a statistical point of view.

Table 11: AA data obtained from extractions with Albariño variety and different solvents

| Grape marc initial weight | Solvent | AA (mM Trolox) | |
|---|---|---|---|
| | | Mean | Standard deviation |
| 1g | Ethanol:$H_2O$ (80:20) a pH=1 | 2.52 | 0.04 |
| 10 g | Ethanol:$H_2O$ (80:20) a pH=1 | 2.20 | 0.05 |
| 20 g (macerating) | Ethanol:$H_2O$ (80:20) a pH=1 | 4.15 | 0.03 |
| 20 g | Ethanol:$H_2O$ (80:20) | 5.24 | 0.04 |
| 20 g | Ethanol:$H_2O$ (50:50) | 10.17 | 0.25 |
| 20 g | Augardente | 9.38 | 0.12 |
| 20 g | Butylen glycol: $H_2O$ (65:35) | 7.38 | 0.09 |

Table 12: AA data obtained from extractions with other white grape marc varieties or with seeds

| Grape marc initial weight | Solvent | AA (mM Trolox) | |
|---|---|---|---|
| | | Mean | Standard deviation |
| 20 g | Caíño Blanco | 5.05 | 0.16 |
| 20 g | Godello | 5.73 | 0.43 |
| 20 g | Loureiro | 5.27 | 0.22 |
| 20 g | Treixadura | 5.58 | 0.16 |
| 20 g | Torrontés | 4.32 | 0.28 |
| 20 g | Semillas | 26.68 | 1.42 |

The AA of the extract obtained in Example 2 was equivalent to 65.46 ± 4.20 mM Trolox or to 5310 ppm of Vitamin C. The mean antioxidant activity (n = 5) of the extracts obtained with the larger column was 73.1 ± 13.7 mM Trolox.

The effectiveness in eliminating free radicals was calculated as detailed in the corresponding methodology section. The results for the extract obtained in Example 2, expressed as half maximal inhibitory concentration ($IC_{50}$) was 432 mg GAE/L. The data for the reference synthetic antioxidants were 730 mg/L and 8498 mg/L for BHA and BHT, respectively. Thus, the extract of Example 2 has a relatively effectiveness in eliminating free radicals equivalent to ≈ 20 times BHT and ≈1.7 times BHA, in solutions of equivalent concentration.

**Example 3.2.** Antibacterial activity

**Ejemplo 3.2.1.** Qualitative assessment of the Antibacterial Activity by Inhibition Zones

**Bacterial strains used:**

*Staphylococcus aureus (S. aureus), Bacillus* spp. y *Propionibacterium acnes (P. acnes).*

**Culture media:**

a. Culture media used for *S. aureus* y *Bacillus spp.*

TSB medium: soya broth and triptone
Luria Broth (LB) medium: 1% triptone, 1% NaCl, 0.5% yeast extract.

b. Culture media used for *P. acnes.*
c. Culture media used for anaerobic bacteria: 3% triptone, 2% yeast extract, 0.05% L-cysteine hydrochloride, 0.5% glucose. Autoclave for 30 minutes at 110 ° C and add 2.5% v/v solution of hemin previously sterile-filtered. Hemin solution (for 100 mL): 0.1 g hemin, 4 mL of triethanolamine and 96 mL of distilled water. All culture media were supplemented with 2% agar-agar when solid required.

The antimicrobial activity of the extract against Gram + has been studied. To test the antimicrobial activity of the polyphenolic grape marc extracts, a variation of the standard diffusion technique described by Fattouch et al. was employed [Fattouch, S.; Caboni, P.; Coroneo, V.; Tuberoso, C. I. G.; Angioni, A.; Dessi, S.; Marzouki, N.; Cabras, P. Antimicrobial activity of Tunisian quince (Cydonia oblonga Miller) pulp and peel polyphenolic extracts. J. Agric. Food Chem. 2007, 55, 963-969.]. The diffusion assays in disk/well are based on the ability of many antimicrobial agents to diffuse into the agar creating a continuous concentration gradient.

If the disk containing the antimicrobial substance is placed onto a newly inoculated plate, the growth inhibition of the analyzed microorganism will occur in a gradient point and will result in a growth inhibition area concentric with the disc/well. This area, being proportional to the minimum inhibitory concentration (MIC) will allow the distinction of sensitive, resistant, or intermediate strains.

A bacterial suspension of *S. aureus* grown in TSB culture medium, and adjusted to a turbidity corresponding to 0.5 in McFarland standard (108 CFU/ml) was the starting point. Superficially, and with the help of a handle Digralsky, an agar plate with a M9 minimal medium was inoculated with 100 mL of the above bacterial suspension in order to obtain a microbial lawn. Then, equidistant wells were made in the agar, where volumes of 100 $\mu$L of the extract obtained as described in Example 1 were pipetted. Chloramphenicol (1000 $\mu$g/mL) was used as the positive control and the extract solvent as the negative control. After 24h incubation, the inhibition zone size was measured using a ruler with a precision of 0.5 mm.

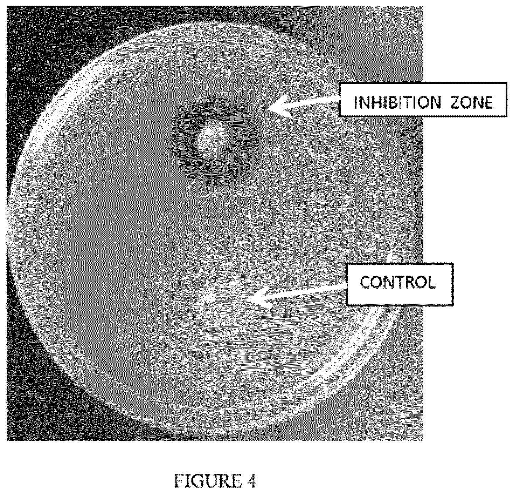

In Figure 4, the photo of the resistance-sensitivity test to the antimicrobial agent is shown, where can be observed how in a plate completely covered by *S. aureus,* appears the "growth inhibition zone" generated by the concentrated extract obtained as described in Example 1. In the center of the plate, a control carried out with 50/50 aqueous ethanol (elution solvent of the extracts) can be seen and where the inhibitory zone is not presented.

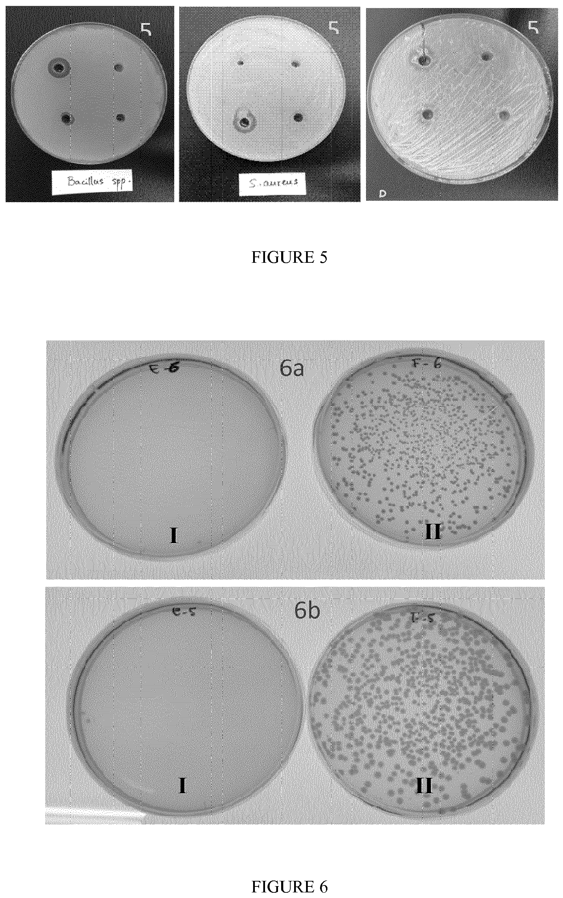

For the *Bacillus spp* assay, it was proceeded similarly, but started from a grown bacterial suspension in LB. In Figure 5, the photos of the resistance-sensitivity test to the antimicrobial agent are shown, where can be observed, how in a plate completely covered by *Bacillus spp.* (figura 5a), appears the "growth inhibition zone" generated by the concentrated extract obtained as described in Example 2; and at two different dilutions, being the diameter of the inhibition zone proportional to the concentration of the extract. Two controls are also shown for the aqueous butylene glycol (65/35) (elution solvent of the extracts) and where no inhibition halo appears. The figure 5b follows the same treatment and results in a plate completely covered by *S. aureus,* in this case also grown in LB medium. The figure 5c is an equivalent test for *P. acnes,* complying with the peculiarities of this anaerobic bacterium culture.

**Example 3.2.** Quantitative assessment of Antibacterial Activity: Determination of the Minimum Inhibitory Concentration (MIC).

**Grown conditions:**

Bacteria were grown in LB medium at 37°C. Liquid cultures were incubated in an orbital shaker at 200 rpm. In the case of *P. acnes,* because it is a strict anaerobic bacterium, the cultures were introduced in an anaerobiosis jar with Anaerocult®A packets (Merck). The anaerobiosis jar was set at an orbital shaker and *P. acnes* was incubated with gentle shaking in liquid medium to ensure the homogeneous distribution of the extract to be tested, since this has a tendency to precipitate when added.

**Example 3.2.1. MIC and influence of the vehicle.**

To determine the minimum inhibitory concentration and the influence of the vehicle (butylene glycol) on growth inhibition, Erlenmeyer flasks were prepared containing 18 mL of liquid culture medium to which different volumes of the extract were added (from 20 $\mu$L to 2 mL), adding the amount of vehicle needed to achieve a final volume of 20 mL in the Erlenmeyer flasks. Thus, the butylene glycol concentration was 6.5% in all cases and the concentration of the extract was tested between 0.1 and 10% v v. These flasks were inoculated with 100 $\mu$L of a culture of the microorganism to be tested in stationary phase.

In each case the following controls were performed:

1. Positive control: microorganism in a culture medium without extract or vehicle
2. Control of the influence of the vehicle: microorganism in a culture medium with 2 mL of vehicle (butyleneglycol:water 65:35)
3. Vehicle contamination control: culture medium with 2 mL of vehicle
4. Extract contamination control: culture medium with 2 mL of extract

Cultures were incubated overnight (aprox. for 14 to 16 hours) in the above conditions. Once the incubation is completed, serial dilutions from them in tubes containing saline solution were performed. From dilutions $10^{-3}$, $10^{-4}$ y $10^{-5}$ 20 $\mu$L of simple were taken and sown by extension in Petri plates with corresponding solid culture medium and incubated for 24 hours at the appropriate temperature. All assays were performed in triplicate. The results were evaluated by the method of CFU count (Colony-Forming Units) on plate.

**Example 3.2.2. MIC in Gram positive bacteria.**

After performing preliminary tests and proven non-vehicle influence on the growth inhibition of Gram positive bacteria, proceeded to more accurately adjust the MIC for *S. aureus, Bacillus spp* and *P. acnes,* using as reference values the ones obtained in the previous study. Thus, various concentrations of the extract between 1 and 3% v/v in 20 mL Erlenmeyer flasks containing the corresponding culture medium were tested. The incubation procedure and plating was identical to those described above. Figures 6a and 6b show the results obtained for *S. aureus* and *Bacillus spp,* respectively.

MIC values for the bacteria studied were as follows:

*S.aureus:* 1.25-1.5 %
*Bacillus spp:* 1-1.25 %
*P. acnes:* 1-1.5 %

This indicates that small dilutions of the extract, between 1 and 1.5 mL in 100 mL of medium could inhibit the visible growth of the microorganisms after incubation.

**Example 3.3.** Protective activity against UV radiation (UV light absorbing activity)

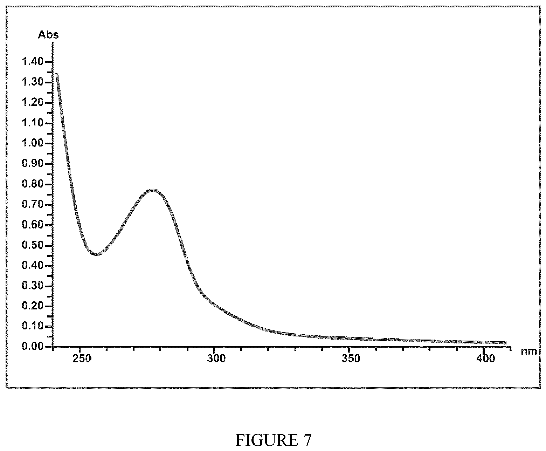

The absorbing activity of the liquid extract obtained as detailed in Example 2 against UV radiation type A and B has been studied. Specifically, the full UV spectrum from 200 to 400 nm, of a 1:500 dilution of the extract, was measured; which is shown in Figure 7. Additionally, Table 13 shows the absorbance values at two wavelengths representative of UVB radiation (280 to 320 nm) and two absorbance values at two wavelengths representative of UVA radiation (350 to 400 nm). The spectral characteristics of the extract, make it useful in potential protective actions against UVB radiation, since it is in this zone where has its maximum absorbance ($\lambda_{max}$ = 277 nm).

Table 13: Absorbance in the UV spectral region of a 1:500 dilution of the extract obtained in Example 2

| | $UV_B$ | | $UV_A$ | |
|---|---|---|---|---|
| Wavelenght (nm) | 280 | 320 | 350 | 400 |
| Absorbance | 0.754 | 0.080 | 0.042 | 0.022 |

**Example 4. Evaluating the stability of the obtained extracts.**

The stability of the extract obtained in Example 2 in different storage conditions was studied and a solution of ascorbic acid at 1% in 65 % butylene glycol was use as *control:*

- Storage temperature (with $N_2$ in the headspace and darkness): Ambient temperature (25 °C); Freezing (-20°C ); Refrigeration (4°C )
- Presence of oxygen (darkness and 25 °C): Vial with $O_2$ in the headsapce
- Exposure to Light ($N_2$ in the headspace and 25 °C): Vial exposed to light.

The controls are scheduled as follows: a weekly check for the first month; a fortnightly monitoring during 2nd month; and, from this moment, a monthly control.

The measured variables were the TP, the AA, the absorbance at λ = 420, 520 and 620 nm; and the concentration of individual identified polyphenols determined by chromatographic analysis. Additionally, the visual appearance of the extracts in each control performed was assessed, showing only a slight increase of color in the vial stored in the presence of $O_2$, which however did not correspond to any significant variation in polyphenol content of the extract or its antioxidant activity. Table 14 summarizes the results obtained. A statistical comparison of the data was assessed, where a *p value* > 0.05 indicates no significant differences; and therefore, the extract is stable in relation to the evaluated parameter.

Table 14: Statistical data of the stability study of the extract of Example 2

|  | Light/ Darkness | | $O_2/ N_2$ | | 25°/4°/-20°C | |
| --- | --- | --- | --- | --- | --- | --- |
| EXTRACT | t[1] | p[2] | t[1] | p[2] | F[3] | p[2] |
| TP | -0.47 | 0.64 | 0.64 | 0.52 | 5.52 | *0.0058* |
| AA | 0.10 | 0.92 | -0.13 | 0.90 | 0.73 | 0.48 |
| Gallic acid | -0.38 | 0.71 | 2.27 | *0.032* | 7.02 | *0.0025* |
| Catechuin | 1.36 | 0.18 | -1.19 | 0.24 | 5.02 | *0.0115* |
| Epicatechuin | 0.13 | 0.89 | -1.16 | 0.26 | 1.83 | 0.17 |
| Quercetins | -0.10 | 0.92 | -0.87 | 0.39 | 1.28 | 0.29 |
| λ=420nm | 1.14 | 0.26 | 2.94 | *0.0076* | 1.26 | 0.30 |
| λ=520nm | -0.78 | 0.44 | 2.62 | *0.015* | 2.59 | 0.09 |
| λ=620nm | -0.47 | 0.64 | 3.57 | *0.0017* | 4.85 | *0.0143* |
| Control (Ascorbic) | 0.065 | 0.95 | -1.56 | 0.126 | 0.07 | 0.93 |

[1]*Student's t* value. parameter used to assess statistically significant differences between two groups of data; null hypothesis ($H_0$): there is no difference between means, versus alternative hypothesis ($H_1$): there is a difference between the means of the two groups of data.
[2]Rejection probability of hypotheses: the threshold value is 0.05 (working at 95% confidence): If *p*>0.05 $H_0$ is accepted; if *p*<0.05 $H_1$ is accepted.
[3]*Fisher's F* value: The F-test of the analysis of variance (ANOVA) determines if there are significant differences between the means of more than two groups of data. If the *p-value* of F-test is less than 0.05, there is a statistically significant difference between the mean of the 3 variables with a level of 95.0% confidence; and vice versa.

The analysis of stability data for the first 100 days of storage of the extract in the different conditions mentioned, it is concluded that the extract is completely stable over this period in relation to the antioxidant activity and the concentration of epicatechin and quercetins. Both catechuin and gallic undergo slight changes in concentration (gallic increases slightly in the presence of $O_2$ and 25 °C) probably from the existence of epicatechin-gallate in the extract and its possible transformation into simple polyphenols. In relation to the absorbance at different wavelengths, the data only vary when the extract is stored at 25 °C in the presence of $O_2$; in such conditions the absorbance increases slightly. Finally, the TP of the extract remains completely stable during the studied period when it is stored in a refrigerator or freezer, decreasing slightly from the first month of storage if stored at room temperature, in the presence of light and with $O_2$; in any case the antioxidant activity of the extract is not affected in any circumstances. The use of nitrogen in storage is not considered necessary, as it is not essential to keep it in the absence of light; although an amber bottle can best retain their initial color characteristics (absorbance at 420, 520 and 620 nm) in the presence of $O_2$.

**Claims**

1. Method to obtain an extract from white grape by-products comprising:

   e) preparing a mixture comprising a white grape marc as a white grape by-product and sand as dispersant,
   f) adding a solvent wherein the solvent is selected independently from the group consisting of water, alkyl alcohol, glycols, or mixtures thereof,
   g) eluting, and
   h) collecting the eluates,

   wherein the weight ratio of the white grape marc and the dispersant is between 1:1 and 1:3.

2. The method according to the preceding claim, comprising:

   a") preparing a mixture comprising white grape marc as the white grape by-product and sand as a dispersant in a weight ratio of between 1:1 and 1:3,
   b") adding a mixture of glycols/water as a dissolvent in a volume of between 0.1 and 5 relative to the weight of the mixture of white grape by-product and dispersant in the step a") and keeping between 5 and 24 hours at a temperature between 10 °C and 40 °C,
   c") eluting,
   d") collecting the eluates,
   e") eluting the mixture of the step a) with the eluates collected in step d').

3. The method according to any of the preceding claims, which further comprises a lyophilization step.

4. The method according to any of the preceding claims, wherein the white grape by-product is grape marc of the variety selected from the group consisting of Albariño, Caíño Blanco, Godello, Torrontés and Treixadura.

5. White grape polyphenolic extract obtainable by a method described in any of the claims 1-4 comprising a total polyphenol index of between 8 and 50 mg of gallic acid/g dry grape marc ; gallic acid concentration of between 0.035 and 0.125 mg/g dry grape marc; catechin concentration of between 0.3 and 1.28 mg/g dry grape marc; epicatechin concentration of between 0.3 and 0.80 mg/g dry grape marc; pH between 4 and 8; and absence of anthocyans .

6. Polyphenolic extract according to claim 5,
   which is in solution, in suspension
   or lyophilized.

7. Polyphenolic extract according to any of the claims 5 and 6 for the treatment of
   bacterial infections, or for the treatment of acne.

8. Cosmetic composition comprising the extract according to any of claims 5 and 6.

9. Pharmaceutical composition comprising the extract according to any of claims 5 and 6.

10. Nutritional composition comprising the extract according to any of claims 5 and 6.

11. Use of the extract as described in any of claims 5 and 6,
    to prepare a food
    preservative.

12. Anti-acne composition comprising the extract described in any of claims 5 and 6.

13. Package or biodegradable packaging comprising the extract described in any of claims 5 and 6.

14. Food preservative comprising the extract described in any of claims 5 and 6.

**Patentansprüche**

1. Verfahren zur Gewinnung eines Extrakts aus Nebenprodukten weißer Trauben, umfassend:

   e) Herstellen eines Gemischs, umfassend einen Trester aus weißen Trauben als ein Nebenprodukt weißer Trauben und Sand als Dispergiermittel,
   f) Zugeben eines Lösungsmittels, wobei das Lösungsmittel unabhängig aus der Gruppe, bestehend aus Wasser, Alkylalkohol, Glykolen oder Mischungen davon, ausgewählt wird,
   g) Eluieren, und
   h) Sammeln der Eluate,

   wobei das Gewichtsverhältnis des Tresters weißer Trauben und des Dispergiermittels zwischen 1:1 und 1:3 liegt.

2. Das Verfahren nach dem vorangehenden Anspruch, umfassend:

   a") Herstellen eines Gemischs, umfassend Trester aus weißen Trauben als das Nebenprodukt weißer Trauben und Sand als ein Dispergiermittel in einem Gewichtsverhältnis zwischen 1:1 und 1:3,
   b") Zugeben eines Glykol-Wasser-Gemischs als Lösungsmittel in einem Volumen zwischen 0,1 und 5 bezogen auf das Gewicht des Gemischs aus dem Nebenprodukt weißer Trauben und Dispergiermittel in dem Schritt a") und Lagerung für 5 bis 24 Stunden bei einer Temperatur zwischen 10 °C und 40 °C,
   c") Eluieren,
   d") Sammeln der Eluate,
   e") Eluieren des Gemischs aus Schritt a) mit den in Schritt d") gesammelten Eluaten.

3. Das Verfahren nach einem der vorangehenden Ansprüche, zusätzlich umfassenden einen Gefriertrocknungsschritt.

4. Das Verfahren nach einem der vorangehenden Ansprüche, wobei es sich bei dem Nebenprodukt aus weißen Trauben um Traubentrester der aus folgender Liste ausgewählten Sorten handelt: Albariño, Caíño Blanco, Godello, Torrontes und Treixadura.

5. Polyphenolextrakt aus weißen Trauben, erhältlich über ein in einem der Ansprüche 1-4 beschriebenen Verfahren, umfassend einen Gesamtpolyphenolindex zwischen 8 und 50 mg Gallussäure/g trockener Traubentrester; eine Gallussäurekonzentration zwischen 0,035 und 0,125 mg/g trockener Traubentrester; eine Catechinkonzentration zwischen 0,3 und 1,28 mg/g trockener Traubentrester; eine Epicatechinkonzentration zwischen 0,3 und 0,80 mg/g trockener Traubentrester; einen pH-Wert zwischen 4 und 8; und die Abwesenheit von Anthocyanen.

6. Polyphenolextrakt nach Anspruch 5, der in Lösung, in Suspension oder gefriergetrocknet vorliegt.

7. Polyphenolextrakt nach einem der Ansprüche 5 und 6 zur Behandlung bakterieller Infektionen oder zur Behandlung von Akne.

8. Kosmetische Zusammensetzung, die das Extrakt nach einem der Ansprüche 5 und 6 enthält.

9. Pharmazeutische Zusammensetzung, die das Extrakt nach einem der Ansprüche 5 und 6 enthält.

10. Nährstoffzusammensetzung, die das Extrakt nach einem der Ansprüche 5 und 6 enthält.

11. Verwendung des in einem der Ansprüche 5 und 6 beschriebenen Extrakts zur Herstellung eines Mittels zur Lebensmittelkonservierung.

12. Anti-Akne-Zusammensetzung, die das Extrakt nach einem der Ansprüche 5 und 6 enthält.

13. Verpackung oder biologisch abbaubare Verpackung, die das Extrakt nach einem der Ansprüche 5 und 6 enthält.

14. Mittel zur Lebensmittelkonservierung, das das Extrakt nach einem der Ansprüche 5 und 6 enthält.

**Revendications**

1. Procédé pour l'obtention d'un extrait à partir de sous-produits de raisin blanc comprenant:

   e) préparer un mélange comprenant un marc de raisin blanc en tant que sous-produit de raisin blanc et du sable en tant que dispersant,
   f) ajouter un solvant dans lequel le solvant est choisi indépendamment parmi le groupe consistant en eau, alcool alkyle, glycols, ou des mélanges de ceux-ci,
   g) éluer, et
   h) recueillir les éluats,

   dans lequel le rapport de marc de raisin blanc et de dispersant est situé entre 1:1 et 1:3.

2. Le procédé selon la revendication précédente, comprenant:

   a") préparer un mélange comprenant du marc de raisin blanc en tant que sous-produit de raisin blanc et du sable en tant que dispersant dont le rapport de poids est situé entre 1:1 et 1:3,
   b") ajouter un mélange de glycols/eau en tant que dissolvant en un volume situé entre 0,1 et 5 par rapport au poids du mélange de sous-produit de raisin blanc et de dispersant de l'étape a") et maintenir entre 5 à 24 heures à une température située entre 10 °C et 40 °C,
   c") éluer,
   d") recueillir les éluats,
   e") éluer le mélange de l'étape a) avec les éluats recueillis lors de l'étape d').

3. Le procédé selon l'une quelconque des revendications précédentes, qui comprend également une étape de lyophilisation.

4. Le procédé selon l'une quelconque des revendications précédentes, dans lequel le sous-produit de raisin blanc est un marc de raisin de la variété choisie parmi le groupe consistant en Albariño, Caíño Blanco, Godello, Torrontés et Treixadura.

5. Extrait polyphénolique de raisin blanc pouvant être obtenu par un procédé décrit dans l'une quelconque des revendications 1-4 comprenant un indice polyphénolique total situé entre 8 et 50 mg d'acide gallique/g de marc de raisin sec; concentration d'acide gallique située entre 0,035 et 0,125 mg/g de marc de raisin sec; concentration de catéchine située entre 0,3 et 1,28 mg/g de marc de raisin sec; concentration d'épicatéchine située entre 0,3 et 0,80 mg/g de marc de raisin sec; pH situé entre 4 et 8; et absence d'anthocyanes.

6. Extrait polyphénolique selon la revendication 5, qui est en solution, en suspension ou lyophilisé.

7. Extrait polyphénolique selon l'une quelconque des revendications 5 et 6 pour le traitement d'infections bactériennes, ou pour le traitement d'acné.

8. Composition cosmétique comprenant l'extrait selon l'une quelconque des revendications 5 et 6.

9. Composition pharmaceutique comprenant l'extrait selon l'une quelconque des revendications 5 et 6.

10. Composition nutritionnelle comprenant l'extrait selon l'une quelconque des revendications 5 et 6.

11. Utilisation de l'extrait tel que décrit dans l'une quelconque des revendications 5 et 6, pour préparer un conservateur alimentaire.

12. Composition anti-acné comprenant l'extrait décrit dans l'une quelconque des revendications 5 et 6.

13. Emballage, ou emballage biodégradable comprenant l'extrait décrit dans l'une quelconque des revendications 5 et 6.

14. Conservateur alimentaire comprenant l'extrait décrit dans l'une quelconque des revendications 5 et 6.

FIGURE 1

FIGURE 2

FIGURE 3

FIGURE 4

FIGURE 5

FIGURE 6

FIGURE 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 08026275 B

- US 6238673 B1

### Non-patent literature cited in the description

- *J. Biochem. Biophys. Methods,* 2007, vol. 70, 151-162
- **ZIAKOVA A ; BRANDSTETEROVÁ E ; BLAHOVÁ E.** Matrix solid-phase dispersion for the liquid chromatographic determination of phenolic acids in Melissa officinalis. *Journal of Chromatography A,* 2003, vol. 983, 271-275
- **XIAO HB ; KRUCKER M ; ALBERT K ; LIANG XM.** Determination and identification of isoflavonoids in Radix astragali by matrix solid-phase dispersion extraction and high-performance liquid chromatography with photodiode array and mass spectrometric detection. *Journal of Chromatography A,* 2004, vol. 1032, 117-124
- *Journal of Chromatography A,* 2006, vol. 1129, 14-20
- **KRISTENSON, E.M. ; HAVERKATE, E.G.J. ; SLOOTEN, C.J. ; RAMOS, L. ; VREULS, R.J.J. ; BRINKMAN, U.A.TH.** Miniaturized automated matrix solid-phase dispersion extraction of pesticides in fruit followed by gas chromatographic-mass spectrometric analysis. *J. Chromatogr. A,* 2001, vol. 917, 277-286

- **KRISTENSON, E.M. ; SHAHMIRI, S. ; SLOOTEN, C.J. ; VREULS, R.J.J. ; BRINKMAN, U.A.TH.** Matrix solid-phase dispersion micro-extraction of pesticides from single insects with subsequent GC-MS analysis. *Chromatographia,* 2004, vol. 59, 315-320
- *Polyphenolic content and in vitro antioxidant characteristics of wine industry and other agri-food solid waste extracts*
- **SINGLETON, V. L. ; ROSSI, J. A., JR.** Colorimetry of total phenolics with phosphomolybdic-phosphotungstic acid reagents. *Am. J. Enol. Vitic.,* 1965, vol. 16 (3), 144-58
- **W. BRAND-WILLIAMS ; M.E. CUVELIER ; C. BERSET.** *LWT - Food Sci. Technol.,* 1995, vol. 28, 25
- **FATTOUCH, S. ; CABONI, P. ; CORONEO, V. ; TUBEROSO, C. I. G. ; ANGIONI, A. ; DESSI, S. ; MARZOUKI, N. ; CABRAS, P.** Antimicrobial activity of Tunisian quince (Cydonia oblonga Miller) pulp and peel polyphenolic extracts. *J. Agric. Food Chem.,* 2007, vol. 55, 963-969